# EUROPEAN PATENT SPECIFICATION

(11) **EP 1 037 653 B1**
(45) Date of publication and mention of the grant of the patent: **06.09.2006**
(21) Application number: 98962052.1
(22) Date of filing: 10.12.1998
(51) Int. Cl.: A61K 38/08, A61K 38/48, A61K 38/55, A61K 38/17, A61K 38/05, A61K 9/127, A61K 8/00

(54) **COMPOSITIONS AND METHODS FOR REGULATING PHAGOCYTOSIS AND ICAM-1 EXPRESSION**
ZUSAMMENSETZUNGEN UND METHODEN ZUR REGULIERUNG DER PHAGOZYTOSE UND ICAM-1 EXPRESSION
COMPOSITIONS ET PROCEDES DE REGULATION DE LA PHAGOCYTOSE ET DE L'EXPRESSION DE L'ICAM-1

(30) Priority: 16.12.1997 US 69797 P
(43) Date of publication of application: 27.09.2000
(73) Proprietor: JOHNSON & JOHNSON CONSUMER COMPANIES, INC., Skillman, NJ 08558 (US)
(72) Inventor: MIRI, Seiberg, Princeton, NJ 08540 (US); SHAPIRO, Stanley, S., Livingston, NJ 07039 (US); EISINGER, Magdalena, Demarest, NJ 07627 (US)
(74) Representative: Fisher, Adrian John
(86) International application number: PCT/US1998/026255
(87) International publication number: WO 1999/030729

(56) References cited:
- EP-A- 0 421 021
- WO-A-96/24371
- C.K. DERIAN ET L.: "DIFFERENTIAL REGULATION OF HUMAN KERATINOCYTE GROWTH AND DIFFERENTIATION BY A NOVEL FAMILY OF PROTEASE-ACTIVATED RECEPTORS." CELL GROWTH & DIFFERENTIATION, vol. 8, no. 7, July 1997, pages 743-749, XP002100833 BALTIMORE, MD, US cited in the application
- R.J. SANTULLI ET AL.: "EVIDENCE FOR THE PRESENCE OF A PROTEASE-ACTIVATED RECEPTOR DISTINCT FROM THE THROMBIN RECEPTOR IN HUMAN KERATINOCYTES." PROCEEDINGS OF THE NATIONAL ACADEMY OF SCIENCES OF USA, vol. 92, September 1995, pages 9151-9155, XP002100834 WASHINGTON US
- M. MOLINO ET AL.: "INTERACTIONS OF MAST CELL TRYPTASE WITH THROMBIN RECEPTORS AND PAR-2" JOURNAL OF BIOLOGICAL CHEMISTRY, vol. 272, no. 7, 14 February 1997, pages 4043-4049, XP002094579 BALTIMORE, MD, US

## Description

### Field of the Invention

This invention relates to the prevention and treatment of mammalian disorders that are ameliorated by altering phagocytosis or ICAM-1 expression in certain cells. The invention provides numerous compositions, methods and articles of manufacture, and addresses a considerable range of disorders such as those of skin and the immune and central nervous systems. This invention is based on the discovery of a mechanism for the regulation of phagocytosis and ICAM-1 expression.

### Background of the Invention

### Phagocytosis and ICAM-1 Expression Generally

Phagocytosis is the cellular process of ingestion, and usually of isolation or destruction, of particulate material. In vertebrates, it is a characteristic function of various leukocytes and reticuloendothelial cells. Phagocytosis serves as an important bodily defense mechanism against infection by microorganisms, and against occlusion of mucous surfaces and tissues by foreign particles and tissue debris. Phagocytosis is distinct from pinocytosis, which is the uptake of fluid by a cell through invagination and pinching off of the plasma membrane. Herein, the terms "phagocytosis" and "cellular ingestion" are used interchangeably.

Intercellular adhesion molecule-1-("ICAM-1") is an inducible cell-surface glycoprotein that is implicated in cell-cell adhesion and phagocytosis. In particular, the regulation of ICAM-1 plays a role in inflammatory situations, septic shock and neurological disorders (reviewed in van de Stolpe and van der Saag, J Mol Med 74:1, 13-33, 1996). ICAM-1 is elevated in autoimmune diseases such as rheumatoid arthritis and psoriasis. Inflammatory and immune responses are impaired in mice deficient in ICAM-1 (Sligh et al., PNAS 90:8529-33, 1993) .

### Mammalian Disorders Related to Phagocytosis and ICAM-1 Expression

The levels of phagocytosis and ICAM-1 expression in different cells have important implications. Numerous examples of these implications are provided here.

### Immune-Related and Inflammatory Disorders

The primary cause of pulmonary emphysema is the accumulation of foreign material (e.g. smoke condensate) in the lung. This accumulation is followed by the recruitment of neutrophils that are degranulated during attempted phagocytosis (Travis, et. al., Am. J. Respir. Crit. Care Med. Vol. 150:5143-5146, 1994).

Immunological lung disorders such as allergic bronchopulmonary aspergillosis cause mucus plugging of airways, eosinophylic pneumonia and bronchiolitis obliterans. In such diseases, neutrophil elastase-cleaved immnoglobulins and digested C3b receptors limit the phagocytosis of pathogens (Greenberger, JAMA, Vol. 278, No.22, 1997). The increase in neutrophil elastase, while impairing phagocytosis, is beneficial for fighting persistent bacterial infections in the lungs, especially in CF patients (Doring, et al., Am. J. Respir. Crit. Care Med. 150:6 Pt 2, S114-7, 1994).

Periodontal diseases start with the accumulation of plaque at the base of the teeth, followed by the growth of opportunistic bacteria below the gum line. As with the immune response in emphysema, neutrophils are recruited to the infected site, followed by their degranulation during frustrated phagocytosis (Travis, et al., Am. J. Respir. Crit. Care Med. Vol. 150:5143-5146, 1994). The rates of adhesion and ingestion of opsonized Staphylococcus Aureus by polymorphonuclear cells ("PMN's") from periodontal patients is significantly reduced relative to healthy controls (MacFarlane, et al., J Periodontol 1992; 63:908-913, 1992).

Individuals who are genetically immuno-compromised, who have acquired immuno-suppression (such as HIV-infected individuals), or who have temporarily acquired immuno-suppression (such as that following organ transplantation, foreign implants, valve replacement or cancer treatment, and the like), often suffer from secondary infections.

Pulmonary polymorphnuclear leukocytes from diabetic patients were shown to have reduced phagocytic activities, both at the level of ingestion and killing of bacteria, compared to healthy individuals (e.g. Musclow, et al, Cytobios, 65:15-24 1991). In particular, diabetic abnormalities in the immune response include impaired chemotaxis, impaired phagocytosis and impaired adhesion (Grant-Theule, Periodontal Abstracts, Vol. 44, No. 3, 1996). These patients often suffer from undesired infections.

### Cardiovascular System Disorders

The formation of atherosclerotic plaques is induced by aging or by restenosis following balloon angioplasty. Atherosclerotic lesions contain cholesterol-rich particles, many of which aggregate and are internalized in an unregulated fashion by macrophage phagocytosis. This phagocytic process is independent of the LDL or scavenger receptor. The lipid-loaded macrophages, called foamy cells, can lead to further growth of the atherosclerotic plaque (Hoff, et al., European Heart Journal, II (Supp. E), 105-115, 1990; Robert, et al., Annals New York Acad. of Sciences, 673:331-341, 1992).

### Central Nervous System Disorders

Microglial cells found at the periphery of amyloid plaque cores have been shown to contain plaque fibrils of beta/A4 amyloid (El Hachimi and Foncin, C.R. Acad. Sci. Paris, Sciences de la vie/Life sciences, 317:445-451, 1994). The ability of microglial cells to phagocytose and clear senile plaque cores is suppressed in the presence of an astrocyte-secreted diffusable factor. This factor prevents the clearance of senile plaques, allowing them to persist in Alzheimer's disease and other neuropathological degenerative processes (DeWitt, et al., Experimental Neurology, 149:329-340, 1998).

Neutrophil phagocytosis was found to be reduced in mentally depressed patients (e.g. McAdams and Leonard, Prog. Neuro-Psychopharmacol. & Biol. Psychiat., Vol. 17:971-984, 1993; Maes et al., J. Psychiat. Res., Vol. 26, No. 2, 125-134, 1992). Patients with phobic disorders have reduced phagocytosis and cell-killing capacities. Benzodiazepine compounds, used in the treatment of neurological disorders, were shown to reduce or inhibit phagocytosis (e.g. Covelli et al., Immunopharmacology and Immunotoxicology, 11(4):701-714, 1989).

### Skin Disorders

Mid-dermal elastosis, a skin disorder, is clinically characterized by the appearance of wrinkles and aged appearance which results, in part, from phagocytosis of morphologically normal elastic tissue (e.g. Fimiani, et al., Arch Dermatol Res., 287:152-157, 1995).

Many types of pigmentation disorders exist in diverse forms. These can be inherited (e.g. vitiligo,), acquired (e.g. post-inflammatory pityriasis alba, idiophatic guttate hypomelanosis, melasma), and transmitted through infection (e.g. tinea versicolor). These disorders can be benign and self-limiting (e.g. isolated café au lait spots, photocontact dermatitis), or a sign of a more serious underlying disease (e.g. multiple café au lait spots, malignant acanthosis nigricans) (Hacker, Postgrad Med 99:177-86, 1996).

UV irradiation is known to induce an inflammatory condition and an abnormal regulation of ICAM-1 expression. This induction has been documented in the form of sunburns and side effects of PUVA therapy. PUVA therapy is used for numerous skin disorders such as psoriasis, a disease associated with upregulation of ICAM-1 expression (e.g. Tronnier, et al., J. Cutan Pathol 1997, 24:278-85; Ahrens, et al., PNAS 1997, 94:6837-41). differentiation has been- recently described by Derian et al., Cell Growth & Differentiation 8:743-749, 1997.

### Summary of the invention

The present invention relates to the use of a serine protease inhibitor for the manufacture of a medicament for treating a mammal afflicted with a disorder which is ameliorated by a decrease in phagocytosis or ICAM-1 expression in appropriate cells, said disorder being an immune system disorder, an inflammatory disorder, a disorder of the central nervous system, a skin disorder, a physical wound or a respiratory disorder.

According to a further aspect, the invention also provides soybean trypsin inhibitor, for the treatment of a mammal afflicted with a disorder which is ameliorated by a decrease in phagocytosis or ICAM-1 expression in appropriate cells, said disorder being an immune system disorder, an inflammatory disorder, a disorder of the central nervous system, a skin disorder, a physical wound, a respiratory disorder, or restenosis.

### Brief Description of the Figures

Figure 1 shows primary keratinocytes exposed to fluorescent microspheres following treatment with Compound I or SLIGRL.
Figure 2 shows cells of a keratinocyte cell line exposed to fluorescent microspheres following treatment with Compound I or SLIGRL.
Figure 3 shows cells of a fibroblast cell line exposed to fluorescent microspheres following treatment with soybean trypsin inhibitor ("STI") or SLIGRL.
Figure 4A shows a dose-response graph of macrophages treated with STI and exposed to fluorescent *E. Coli.*
Figure 4B shows a dose-response graph of macrophages treated with Compound I or SLIGRL and exposed to fluorescent *E. Coli.*
Figure 5A shows melanin ingestion by keratinocytes treated with SLIGRL, STI or Compound I.
Figure 5B shows the same results as in Figure 5A using isolated melanosomes.
Figure 6A shows ICAM-1 immuno-fluorescence staining of treated keratinocytes.
Figure 6B shows a Western blot of immuno-precipitated ICAM-1 protein from treated keratinocytes.
Figure 7A shows human skin, grafted on immuno-suppressed mice, and treated with vehicle or SLIGRL.
Figure 7B shows histological sections of human skin, grafted on immuno-suppressed mice, and treated with vehicle or SLIGRL.
Figure 7C shows histological sections of human skin, grafted on immuno-suppressed mice, and treated with vehicle or STI.
Figure 8 shows scanning electron microscopy images of treated keratinocytes.
Figure 9 shows F-actin staining of treated keratinocytes.
Figure 10 shows the effect of anti-ICAM-1 antibodies on keratinocyte phagocytosis.
Figure 11 shows the effect of compounds of this invention in lightening human age spots.

### Detailed Description of the Invention

This invention is based on the discovery that PAR2-mediated phagocytosis and PAR-2 mediated ICAM-1 expression can be specifically altered. This ability to specifically increase and decrease these cellular functions permits the treatment and prevention of disorders, which would be ameliorated by an increase, or decrease of phagocytosis and/or ICAM-1 expression.

Accordingly, this invention provides the use of a serine protease inhibitor for the manufacture of a medicament for treating a mammal afflicted with a disorder which is ameliorated by a decrease in phagocytosis or ICAM-1 expression in appropriate cells, said disorder being an immune system disorder, an inflammatory disorder, a disorder of the central nervous system, a skin disorder, a physical wound, a respiratory disorder, or restenosis.

According to a further aspect, the invention also provides soybean trypsin inhibitor, for the treatment of a mammal afflicted with a disorder which is ameliorated by a decrease in phagocytosis or ICAM-1 expression in appropriate cells, said disorder being an immune system disorder, an inflammatory disorder, a disorder of the central nervous system, a skin disorder, a physical wound, a respiratory disorder, or restenosis.

Compositions useful in the practice of the present invention can be of any form known in the art. In one embodiment, the composition comprises a pharmaceutically acceptable carrier and one or more discrete pharmaceutical compounds that function as a serine protease inhibitor. In another embodiment, the composition comprises a naturally-occurring composition, or an extract or component thereof, which is deemed pharmaceutically or cosmetically acceptable. Such naturally occurring compositions contain certain components which function as a serine protease inhibitor, and numerous others that serve as pharmaceutically or cosmetically acceptable carriers. The compositions can be artificial, naturally occurring, or a combination thereof. In addition, the compositions can be of any physical form known in the art, such as liquids (e. g., solutions, creams, lotions, gels, injectables), solids (e. g., tablets, capsules, powders, granules), aerosols, and coatings.

Natural compounds that act as serine protease inhibitors, and in particular, soybean trypsin inhibitor ("STI"), can be used for this invention. Soybean extracts, limabean extracts and similar extracts, and other natural products made from soybean and the like, such as soybean milk, soybean paste, miso, trypsin inhibitor from soybean or limabean and the like, can also reduce phagocytosis by this mechanism. In the preferred embodiment, the naturally occurring composition is soy milk or STI. Additional sources- of serine protease inhibitors include, for example, the following plant families: Solanaceae (e.g., potato, tomato, tomatilla, and the like); Gramineae (e.g., rice, buckwheat, sorghum, wheat, barley, oats and the like); Cucurbitaceae (e.g., cucumbers, squash, gourd, luffa and the like); and, preferably, Leguminosae (e.g., beans, peas, lentils, peanuts, and the like).

As an example, formulations can contain soybean milk or other liquid formulations derived directly from legumes or other suitable plant. In one example, such a formulation contains a large proportion of soybean milk, an emulsifier that maintains the physical stability of the soybean milk, and optionally, a chelating agent, preservatives, emollients, humectants and/or thickeners or gelling agents.

The serine protease inhibitor can be any type of compound known in the art.

In the preferred embodiment for decreasing phagocytosis, the agent is a soybean derivative (such as soybean milk, soybean paste or STI) or Compound I. Compound I has the chemical formula (S)-N-Methyl-D-phenylalanyl-N-[4-[(aminoiminomethyl)amino]-1-(2-benzothiazolylcarbonyl)butyl]-L-prolinamide (as identified in Chemical Abstracts), and has the structure shown below.

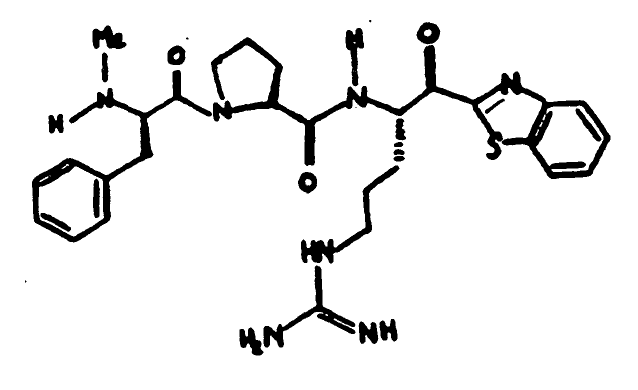

This compound is described in U.S. Patent No. 5,523,308, as well as in Costanzo, et al., J. Med. Chem., 1996, 39:3039-3043. U.S. Patent No. 5,523,308 describes related compounds that behave as serine protease inhibitors (such as compounds with a d-phenylalanine-proline-arginine motif), and that can therefore be used to decrease phagocytosis and ICAM-1 expression. Additional compounds related to Compound I are described in detail in the Examples below.

As used herein, the term "mammal" means any member of the higher vertebrate animals included in the class Mammalia, as defined in Webster's Medical Desk Dictionary 407 (1986), and includes but is not limited to humans, other primates, pigs, dogs, and rodents (such as immuno-suppressed mice). In the preferred embodiment of this invention, the mammal is a human.

Disorders that can be treated or prevented using the instant invention include immune system disorders, inflammatory disorders, disorders of the central nervous system, skin disorders, physical wounds, and respiratory disorders.

A number of disorders have characteristics of more than one category of disorder. Such disorders include, for example, adhesion disorders, which can be categorized as both skin disorders and immune system disorders. Accordingly, a statement herein that a disorder is of a particular category (e.g., skin disorder) means that, at the very least, the disorder bears traits of that category. Again, however, the disorder may additionally bear traits of another category.

Increasing the ability of immune cells to ingest foreign objects like bacteria and viruses would be expected to enhance the immune response. For example, mononuclear phagocytes are inactive in chronic microbial infections (Reiner, Immunol Today 15:8, 374-81, 1994), and their re-activation would be expected to treat the disease. Conversely, disorders wherein the immune system is too active would be ameliorated by inhibiting phagocytosis.

Immune system and inflammatory disorders treatable in this invention include, by way of example, asthma, damage due to toxic substance exposure (e.g., asbestos or smoke), host rejection of implants and transplanted tissue, adhesion disorders, mild infections (such as common colds), severe infections (such as meningitis or "killer bacteria"), wounds (such as infected, diabetic, acute and chronic wounds), restenosis, cystic fibrosis, pulmonary emphysema, and diaper rash.

Skin disorders include unwanted pigmentation, unwanted de-pigmentation, psoriasis, rashes, and certain physical skin imperfections (e.g., wrinkles). In one specific example, vitiligo patients are treated with Compound I to lighten the darker sites Central nervous system disorders include, without limitation, depression, phobic disorders, and other disorders resulting from secondary effects of benzodiazepine treatment.

The mammalian cells treated in the instant methods are preferably PAR-2-expressing cells, and include, without limitation, keratinocytes, fibroblasts, and "professional phagocytes" (i.e., cells having phagocytosis as a primary function). Professional phagocytes include, for example, neutrophils, macrophages and macrophage-like cells (e.g., Langerhans cells and Kupfer cells). In the preferred embodiment, the mammalian cells are human cells.

In this invention, the "appropriate cells" in which phagocytosis or ICAM-1 expression must be altered in response to the instant compositions of matter are readily determined based on the nature of the disorder being treated or prevented. For example, if the disorder being treated is a pigmentation disorder, the appropriate cells in which phagocytosis or ICAM-1 expression needs to be altered are keratinocytes.

The instant methods are directed at preventing as well as treating disorders. As used herein, "treating" a disorder means reducing the disorder's progression, ceasing the disorder's progression, ceasing or otherwise ameliorating secondary effects of the disorder, reversing the disorder's progression, or preferably, curing the disorder. As used herein, "preventing" a disorder means reducing, and preferably eliminating, the likelihood of the disorder's occurrence.

In this invention, administering the instant compositions can be effected or performed using any of the various methods and delivery systems known to those skilled in the art. The administering can be performed, for example, intravenously, orally, via implant, transmucosally, topically, transdermally, intramuscularly, subcutaneously, and via aerosol. In addition, the instant compositions ideally contain one or more routinely used pharmaceutically or cosmetically acceptable carriers. Such carriers are well known to those skilled in the art. The following delivery systems, which employ a number of routinely used carriers, are only representative of the many embodiments envisioned for administering the instant composition.

Transdermal delivery systems include patches, gels, tapes, lotions, soaps, shampoos and creams, and can contain excipients such as solubilizers, permeation enhancers (e.g., fatty acids, fatty acid esters, fatty alcohols and amino acids), hydrophilic polymers (e.g., polycarbophil and polyvinylpyrolidone), and adhesives and tackifiers (e.g., polyisobutylenes, silicone-based adhesives, acrylates and polybutene).

Topical delivery of some of the compositions of this invention, particularly those comprising proteins such as trypsin, tryptase and STI, can be achieved using liposomes. The liposomes are preferably non-ionic. In one example, they contain (a) glycerol dilaurate; (b) compounds having the steroid backbone found in cholesterol; and (c) fatty acid ethers having from about 12 to about 18 carbon atoms, wherein the constituent compounds of the liposomes are in a ratio of about 37.5:12.5:33.3:16.7. Liposomes comprising glycerol dilaurate/cholesterol/ polyoxyethylene-10-stearyl ether/polyoxyethylene-9-lauryl ether ("GDL" liposomes) are preferred. In one embodiment, the liposomes are present in an amount, based upon the total volume of the composition, of from about 10 mg/ml to about 100 mg/ml, and preferably from about 15 mg/ml to about 50 mg/ml. A ratio of about 37.5:12.5:33.3: 16.7 is preferred. Methods of preparing liposomes are well known in the art, such as those disclosed in Niemiec, et al., 12 Pharm. Res. 1184-88 (1995).

Also, for topical or transdermal administration, the instant compositions can be combined with other components such as moisturizers, cosmetic adjuvants, anti-oxidants, bleaching agents, tyrosinase inhibitors and other known depigmentation agents, alpha-hydroxy acids, surfactants, foaming agents, conditioners, humectants, fragrances, viscosifiers, buffering agents, preservatives, sunscreens and the like. The compositions of this invention can also contain active amounts of retinoids including, for example, tretinoin, retinol, esters of tretinoin and/or retinol and the like.

Transmucosal delivery systems include patches, tablets, suppositories, pessaries, gels and creams, and can contain excipients such as solubilizers and enhancers (e.g., propylene glycol, bile salts and amino acids), and other vehicles (e.g., polyethylene glycol, fatty acid esters and derivatives, and hydrophilic polymers such as hydroxypropylmethylcellulose and hyaluronic acid).

Injectable drug delivery systems include solutions, suspensions, gels, microspheres and polymeric injectables, and can comprise excipients such as solubility-altering agents (e.g., ethanol, propylene glycol and sucrose) and polymers (e.g., polycaprylactones and PLGA's). Systems for central nervous system delivery include, for example, a lipid-coupled derivative to cross the blood brain barrier (e.g. DHA). Implantable systems include rods and discs, and can contain excipients such as PLGA and polycaprylactone.

Oral delivery systems include tablets and capsules. These can contain excipients such as binders (e.g., hydroxypropylmethylcellulose, polyvinyl pyrilodone, other cellulosic materials and starch), diluents (e.g., lactose and other sugars, starch, dicalcium phosphate and cellulosic materials), disintegrating agents (e.g., starch polymers and cellulosic materials) and lubricating agents (e.g., stearates and talc). Such delivery systems also include, for example, toothpaste, mouthwash, lozenges and lollipops.

Solutions, suspensions and powders for reconstitutable delivery systems include vehicles such as suspending agents (e.g., gums, zanthans, cellulosics and sugars), humectants (e.g., sorbitol), solubilizers (e.g., ethanol, water, PEG and propylene glycol), surfactants (e.g., sodium lauryl sulfate, Spans, Tweens, and cetyl pyridine), preservatives and antioxidants (e.g., parabens, vitamins E and C, ascorbic acid, and natural extracts), anti-caking agents, coating agents, and chelating agents (e.g., EDTA). Oil-in-water emulsions, water-in-oil emulsions, solvent-based formulations and aqueous gels known to those of skill in the art can also be utilized as vehicles for the delivery of the compositions of this invention.

Methods of determining therapeutically and prophylactically effective doses for administering the instant compositions in humans are known in the art. For example, these effective doses can readily be determined from the results of animal studies.

In one example, the instant composition is applied to the skin surface such that, based upon a square cm of skin surface, from about 2 µl/cm² to about 200 µl/cm² of phagocytosis-altering agent is present when a change in phagocytosis is desired. When using a thrombin and trypsin inhibitor such as Compound I or its analogs, whether synthetically- or naturally-derived in a formulation, such an active compound is present in an amount of from about 0.0001% to about 15% by weight/volume of the composition. In another embodiment, it is present in an amount of from about 0.0005% to about 5% of the composition. Preferably, it is present in an amount of from about 0.001 to about 1% of the composition.

In another example, liquid derivatives and natural extracts made directly from plants or botanical sources are employed in the instant compositions in a concentration (w/v) of from about 1 to about 99%, and preferably from about 75 to about 95%. In still another example, fractions of natural extracts and naturally derived protease inhibitors such as STI have a concentration range of from about 0.01% to about 20% and, preferably, from about 1% to about 10% of the composition.

This invention still further provides an article of manufacture for administering to a mammal the instant composition of matter, comprising a solid delivery vehicle having the composition operably (i.e., deliverably) affixed thereto. The solid delivery vehicle can be any device designed to come into temporary or permanent contact with the body, whether or not it was originally intended for use as a delivery vehicle. Examples of the instant article of manufacture include, without limitation, coated bandages or other wound dressing for treating wounds, coated bodily implants (including implants with coated internal scaffolding) for either preventing or promoting tissue growth, and coated balloon catheters and stents for preventing restenosis.

Finally, this invention provides a method of administering a therapeutic, prophylactic or cosmetic compound to a mammal, comprising administering to the mammal (a) the compound and (b) a composition of matter comprising a pharmaceutical or cosmetic carrier and an agent that specifically increases phagocytosis in an amount sufficient to increase phagocytosis in cells where uptake of the compound is desired, wherein the composition is administered prior to and/or concurrently with the administration of the compound.

The pharmaceutical compound can be, for example, a polypeptide, protein, or nucleic acid molecule. In one embodiment, the pharmaceutical compound and composition are administered together via microscopic porous biodegradable beads, which then release the pharmaceutical compound after being ingested through phagocytosis by the appropriate cells.

This invention will be better understood by reference to the Examples which follow, but those skilled in the art will readily appreciate that they are only illustrative of the invention as described more fully in the claims which follow thereafter.

### Examples

### Example 1

### SLIGRL, STI and Compound I Affect Keratinocyte Phagocytosis

In order to study the role of the PAR-2 pathway in phagocytosis, several *in vitro* model systems were used. One system used contained primary human keratinocytes or a human keratinocyte cell line. In this and a number of following examples, cells were treated with test compounds for different amounts of time (from one hour to three days), and samples were then incubated with fluorescent microspheres for two hours. The beads ingested by the cells were photographed using fluorescence microscopy.

In this example, human primary keratinocytes or the human keratinocyte cell line HaCaT were used as *in vitro* model systems to study the effect of PAR-2 regulators on keratinocyte phagocytosis. The human primary keratinocytes used are commercially available from Clonetics (San Diego, CA). Cells were plated on chamber slides, at 2 chambers/slide and 60,000 cells/chamber. Cells were treated once daily, for two or three days, with Compound I (1µM), SLIGRL (10µM), or vehicle (Phosphate-buffered saline, ("PBS") from Gibco-BRL (Gaithersburg, MD). After two or three days of exposure to the test compounds, cells were exposed to Nile-red or FITC fluorescent microspheres, 1 µm in diameter, 50 microspheres/cell, for two hours at 37°C. Microspheres were from Molecular Probes (Eugene, OR), and were processed according to manufacturer's instructions. Following that treatment, cells were incubated with 15% Fetal Bovine Serum ("FBS", from Gibco-BRL), for 15 minutes at 37°C and rinsed with PBS. At that time, chambers were separated from the slides, and the slides were covered with glycerol and coverslips. Fluorescent microscopy was performed using a Zeiss Axiovert 35 or a Nikon Optiphot-2 microscope.

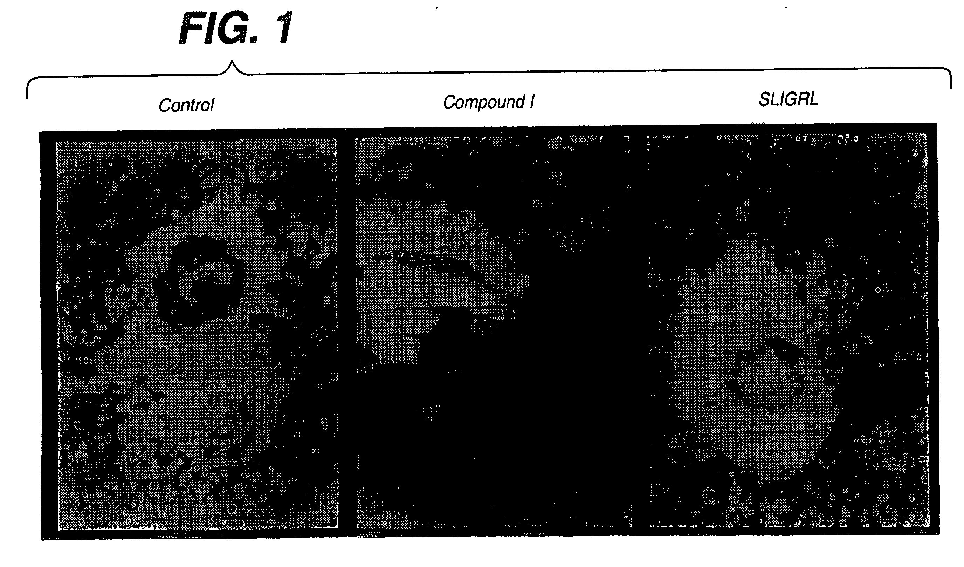

Figure 1 shows three images of human primary keratinocytes, treated for two days with vehicle (control), Compound I or SLIGRL. As seen in this Figure, the microspheres were ingested by the control keratinocytes, and were distributed around the cell's nucleus. Microspheres were also found around the cell, probably because they are non-specifically attached to extracellular-matrix components secreted by the cell. The quantity of the microspheres ingested was changed with the treatments. Treatment with Compound I, an inhibitor of PAR-2 activation results in a dramatic reduction in the quantity of ingested microspheres. Treatment with SLIGRL, a PAR-2-activating peptide, results in a dramatic increase in the number of ingested microspheres.

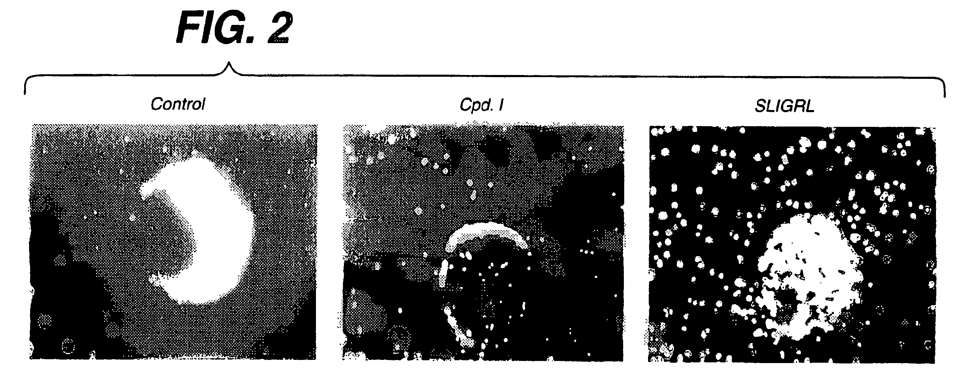

The same results were also obtained when the human keratinocyte cell line HaCaT was used instead of the primary keratinocytes (see Figure 2). SLIGRL, STI and Compound I were tested for their effect on keratinocyte phagocytosis. In these experiments, the extracellular accumulation of microspheres could be washed off. The only particles visible were microspheres internalized by the keratinocytes, which were accumulated around their nuclei. Soybean trypsin inhibitor ("STI"), which is a serine protease inhibitor capable of affecting the PAR-2 pathway, was shown to reduce microsphere ingestion in this experiment, as was Compound I. SLIGRL treatment, on the other hand, resulted in increased microsphere ingestion. Each of these experiments was repeated at least three times. These experiments show, for the first time, that keratinocytes have PAR-2-mediated phagocytic ability. These experiments also demonstrate that compounds that regulate the PAR-2 pathway can regulate the level of keratinocyte phagocytosis.

When these experiments were repeated using melanocytes, which do not express PAR-2, SLIGRL had no inducible effect on microsphere ingestion. Melanocytes did not ingest beads under any of the above conditions. Since SLIGRL activates PAR-2 only, and melanocytes do not express PAR-2, these cells do not respond to the SLIGRL signal and phagocytosis cannot be affected.

### Example 2

### SLIGRL and Compound I Affect Fibroblast Phagocytosis

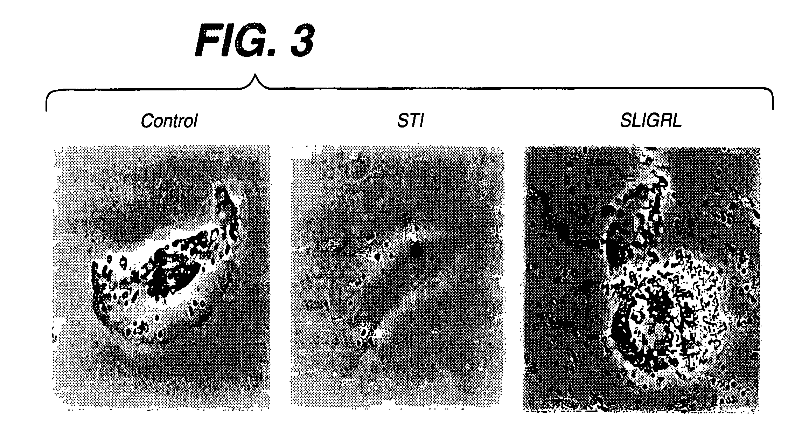

The experiment described in Example 1 was repeated using the fibroblast cell line 92-3T3 (obtained from the ATCC in Rockville, MD). Fibroblasts are not known to posses phagocytic ability. Indeed, only minimal bead ingestion was observed with untreated fibroblasts. However, SLIGRL-treated fibroblasts increased the number of ingested beads (Figure 3). SLIGRL-induced fibroblast phagocytosis was quantitatively different from that of keratinocytes, since fibroblasts do not perform phagocytic tasks in vivo. This experiment shows, for the first time, that fibroblasts have inducible PAR-2 phagocytic ability. In other words, this experiment demonstrates that compounds that regulate the PAR-2 pathway can regulate the level of fibroblast phagocytosis.

### Example 3

### SLIGRL, STI and Compound I Affect Macrophage Phagocytosis

The experiment described in Example 1 was repeated using the macrophage cell line IC-21 (obtained from the ATCC), which shares phagocytic characteristics with peritoneal macrophages. As shown for keratinocytes and fibroblasts, Compound I and STI reduced, and SLIGRL increased, the number of microspheres ingested by these macrophages which are "professional phagocytic" cells.

To better quantify the level of phagocytosis, the "vybrant^{™} Phagocytosis Assay Kit" of Molecular Probes (Eugene, Oregon) was used, following manufacturer's instructions, with modification of cell culture conditions for the IC-21 cell line. This kit uses Fluorescein-labeled *E*. *Coli* K-12 particles, and is designed for quantifying the effects of drugs or other environmental factors on phagocytic functions. Macrophages were treated overnight with 100nM of Compound I, 5µM of SLIGRL, or 0.1 mg/ml of STI, all dissolved in PBS. The ability of the treated macrophages to ingest the fluorescent *E*. *Coli,* as measured by this kit, is documented in Table 1. This experiment was repeated three times. Table 1 represents data from one experiment.

**Table 1**

| Treatment | % effect (ingestion) |
|---|---|
| Untreated control | 100 |
| SLIGRL | 331.6 +/- 5.9 |
| Compound I | 89.9 +/- 13.6 |
| STI | 56.06 +/- 12.4 |

This experiment demonstrates that macrophage phagocytosis can be regulated by PAR-2 pathway modulators. It also shows that both synthetic compounds and naturally derived compounds can modulate phagocytosis via the PAR-2 pathway.

### Example 4

### Dose-response Relationship Between PAR-2 Signaling and Macrophage Phagocytosis

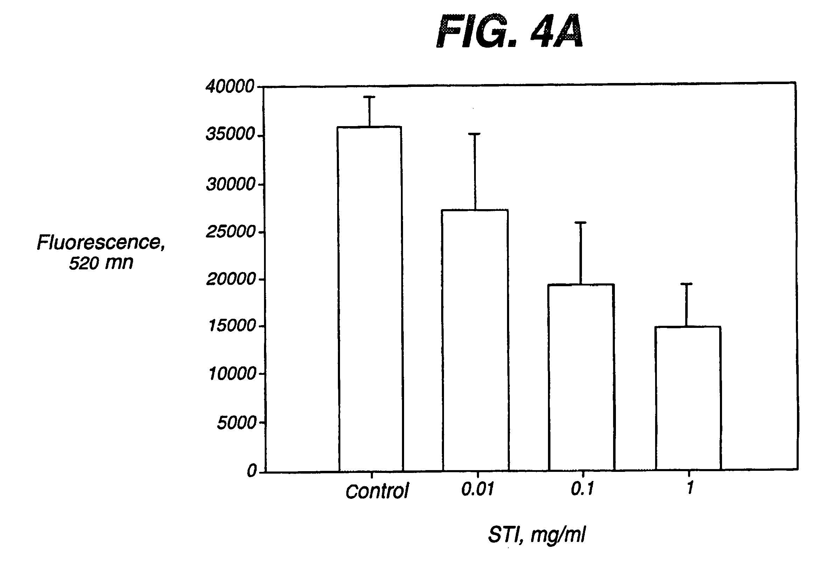

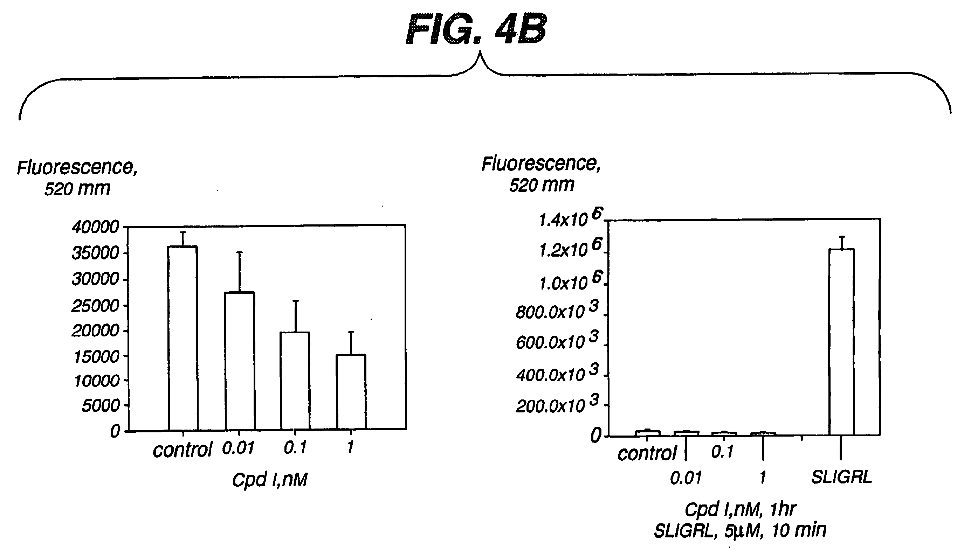

In order to verify the quantitative nature of the macrophage-phagocytosis assay, a dose-response experiment was performed. Macrophages were treated with 0, 0.01, 0.1 and 1 mg/ml of STI, and the experiment was performed as described in Example 3. A dose-response of decreased phagocytosis with increasing STI concentrations was observed, as indicated in Figure 4A. Similar results were obtained for Compound I at 0.01, 0.1 and 1 nM, while SLIGRL treatment resulted in an increase in phagocytosis (Figure 4B). Each experiment was repeated three times. This experiment demonstrates that the phagocytic effect of PAR-2-modulating compounds is dose-responsive and can be quantified.

### Example 5

### Dose-response Relationship Between PAR-2 Signaling and Keratinocyte Phagocytosis

Human keratinocytes were treated with increasing concentrations of SLIGRL, the PAR-2 peptide activator and agonist, at 0, 5 and 10 µM for two days in the same manner as set forth in Example 1. Increasing concentrations of SLIGRL result in increased phagocytosis. Human keratinocytes were also treated with increasing concentrations of Compound I and STI for two days. Treatment with increasing concentrations of Compound I (from 1pM to 1µM) or with STI (from 0.01 to 1 mg/ml), results in a dose-dependent decrease in phagocytosis (see Table 2).

Image analysis of the fluorescent beads inside the keratinocytes was used as an alternative way to quantify the phagocytic effect in this system. Empire Imagins Database Version 1.1 was used on a Gateway 2000 P5-100 computer (Media Cybernetics, Silver Springs, MD) for capturing images. Image Pro Plus version 1.3 was used for measurements, and Microsoft Excel version 5.0 was used for data processing. Data obtained from this keratinocyte-microsphere system were in full agreement with data from the macrophage/*E. Coli* system.

**Table 2**

| Treatment | % Ingestion |
|---|---|
| Untreated | 100 +/- 12 |
| STI, 0.01% | 76 +/- 15 |
| STI, 0.1% | 55 +/- 14 |
| STI, 1% | 41.6 +/- 11 |

### Example 6

### Compound I, SLIGRL and STI Affect the Acquisition of Pigment by Keratinocytes

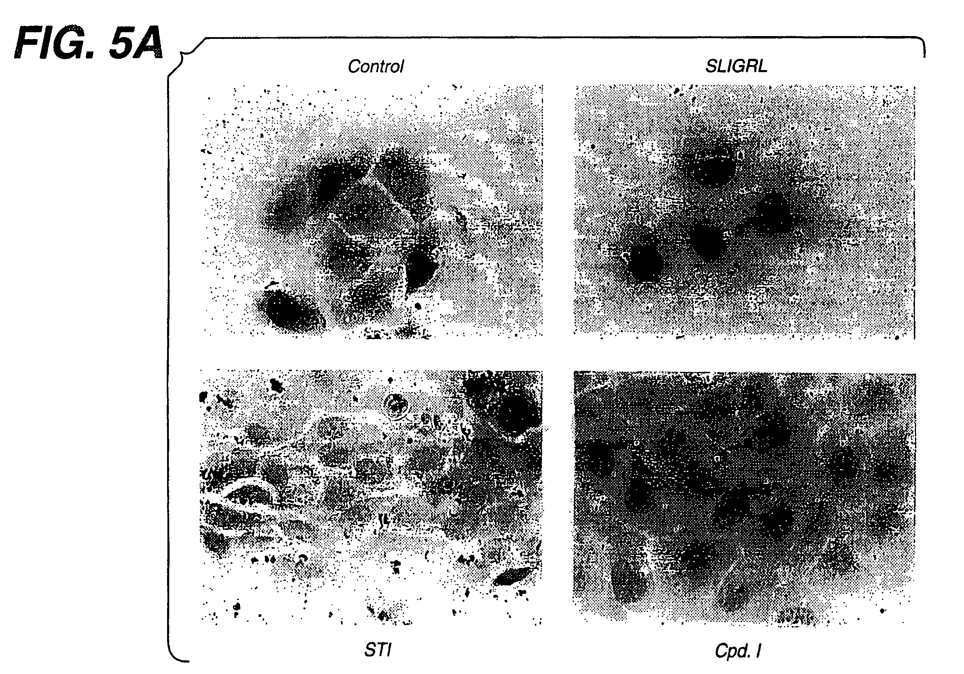

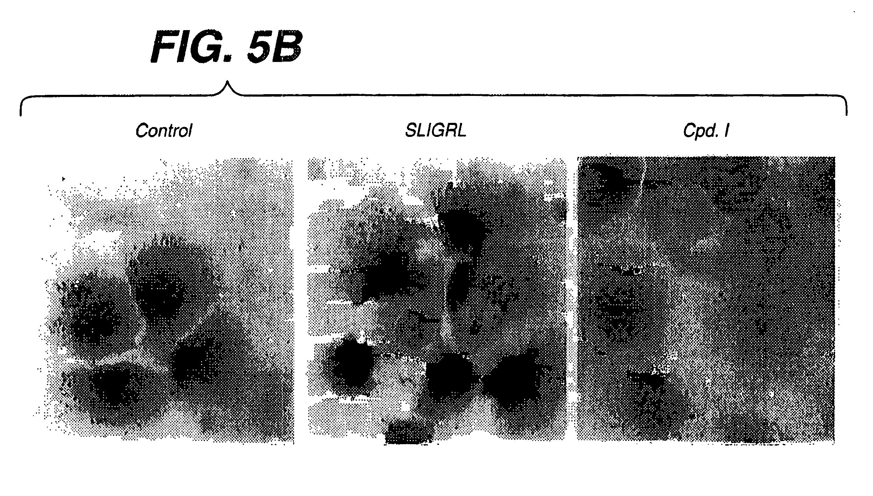

This example tests the ability of keratinocytes to acquire melanin or melanosomes *in vitro,* and thus function as a simplified system for melanosome uptake in the skin. Keratinocytes were plated in glass chamber-slides as described earlier, and were treated for two days with SLIGRL, Compound I or STI. At that time, melanin powder (from Sigma, St. Louis, MO) was mixed in sterile PBS at 10 µg/ml, and was added to the culture media (1:10 dilution) for two hours. Cells were then washed with PBS and stained with Fontana-Mason ("F&M") staining. F&M stains silver nitrate-reducing molecules, thereby permitting the identification of melanins inside the keratinocytes. As shown in Figure 5A, untreated keratinocytes were able to ingest melanin from the culture media, and localize the internalized melanin around their nuclei. This system, therefore, can mimic melanosome transfer and melanin distribution in vivo, as skin keratinocytes use melanin as an UV-protective cap over their nuclei. This capping pattern is also observed with the ingested microspheres as demonstrated in Example 1, and Figures 1 and 2. Figure 5A also shows that the SLIGRL treatment, which turns on the PAR-2 pathway, dramatically increases the internalization of melanin and its deposition around the nuclei. Compound I and STI, on the other hand, dramatically reduce the uptake of melanin by the keratinocytes. This example demonstrates that PAR-2-modulating agents, of both synthetic and natural origin, can affect pigment distribution in epidermal cells. The same results were also observed using melanosomes isolated as described in S. Orlow, et al., J.I.D. 100:55-64 (1993) (Figure 5B).

### Example 7

### Cell-Cell Contact is Required for Compound I Effect on Pigment Transfer from Melanocytes to Keratinocytes.

Since PAR-2 is expressed in keratinocytes, but not in melanocytes, the possible requirement of keratinocyte-melanocyte contact was tested for the effect of Compound I and SLIGRL on melanosome phagocytosis by the keratinocytes. Primary melanocyte cultures (commercially available from Clonetics, San Diego) were plated under epidermal equivalents (EpiDerm, of MatTek, Ashland, MA) to create an equivalent-monolayer co-culture with no contact between keratinocytes and melanocytes. These co-cultures were compared to MelanoDerm equivalents (of MatTek), where melanocytes are present in the basal layer of the equivalent. Cultures were treated with Compound I, with the PAR-2 agonist TFLLRNPNDK, and with the PAR-2 agonist SLIGRL. As set forth in Table 3, keratinocytes are indicated by "K", melanocytes are indicated by "M", and lack of keratinocyte-melanocyte contact is indicated as "no K-M cont". As shown in Table 3, no effect on melanosome transfer was observed in equivalent-monolayer co-cultures (having no keratinocyte/ melanocyte contact) treated with these agents, when measured as the level of pigmentation. In melanocyte-containing equivalents, Compound I reduced and SLIGRL induced pigmentation by affecting melanosome transfer. The same result was also observed with monolayer keratinocyte/melanocyte co-cultures having keratinocyte/melanocyte contact. These results demonstrate that keratinocyte-melanocyte contact is required for the PAR-2 effect on melanosome phagocytosis.

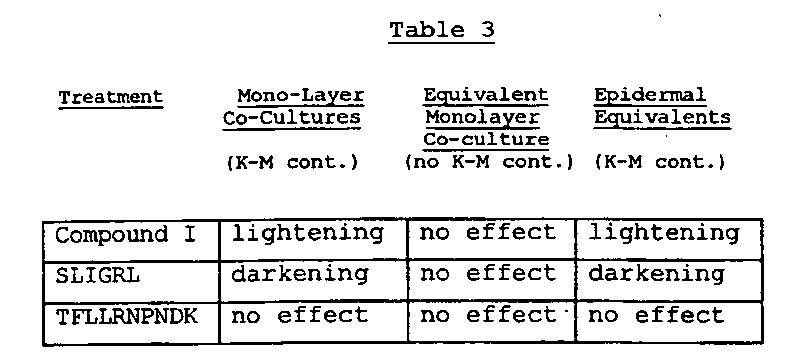

### Example 8

### Timing of the Effect of Compound I and SLIGRL on Phagocytosis

Human keratinocytes were treated with Compound I or SLIGRL for periods of time ranging from one hour to two days. At the end of the treatment period, the cells were treated with microspheres as described in Example 1. Table 4 shows the time required for these compounds to affect phagocytosis. Plus signs indicate an effect on phagocytosis, minus signs indicate no effect on phagocytosis, and plus/minus signs indicate a marginal effect on phagocytosis. The effects measured were a decrease for Compound I and an increase for SLIGRL. This experiment demonstrates that following the activation or the inhibition of the PAR-2-signaling pathway, at least eight hours are required to alter the phagocytic ability of the keratinocytes. This implies that the PAR-2 signaling results in new protein synthesis, a reduction in protein synthesis when a turnover time is required to eliminate the existing relevant protein(s), or a rearrangement of proteins (as occurs in the reorganization of cytoskeletal components).

**Table 4**

| Time of Treatment | Compound I | SLIGRL |
|---|---|---|
| 1 hour | - | - |
| 2 hours | - | - |
| 4 hours | - | - |
| 6 hours | - | - |
| 8 hours | -/+ | -/+ |
| 16 hours | + | + |
| 24 hours | + | + |
| 48 hours | + | + |

### Example 9

### Compound I, STI and SLIGRL Affect ICAM-1 Intracellular Expression and Localization

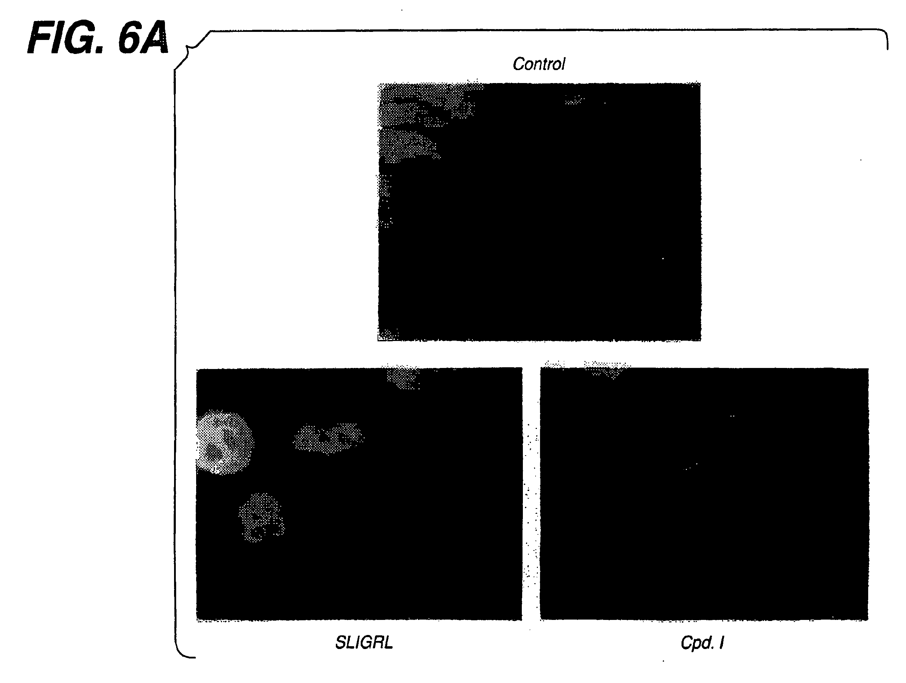

Intercellular adhesion molecule-1 (ICAM-1) is an inducible cell-surface glycoprotein that is implicated in cell-cell adhesion, cell membrane raffling and phagocytosis. Therefore, the effect of PAR-2 modulation on ICAM-1 was tested. Keratinocytes were grown in chamber slides and treated with SLIGRL, Compound I and STI as described, followed by immunofluorescent staining for ICAM-1 using standard procedures. Normal donkey serum, (used at 1:5 dilution), was obtained from Jackson Immunoresearch Laboratory (Westgrove, PA). A polyclonal goat antihuman ICAM-1 antibody, (used at 1:200 dilution), was obtained from R&D Systems (Minneapolis, MN), and FITC-conjugated donkey anti-goat antibody was obtained from Jackson Immunoresearch Laboratory. As shown in Figure 6A, the intracellular localization of ICAM-1 can be modulated via the PAR-2 pathway. In untreated keratinocytes, ICAM-1 was localized mainly to the plasma membrane, with some staining at the nuclear membrane. Following Compound I or STI treatment, less staining was observed at the cytoplasmic membrane, and more at the nuclear membrane. SLIGRL treatment had the opposite effect, resulting in increased and more diffused cytoplasmic membrane staining, and reduced nuclear membrane staining.

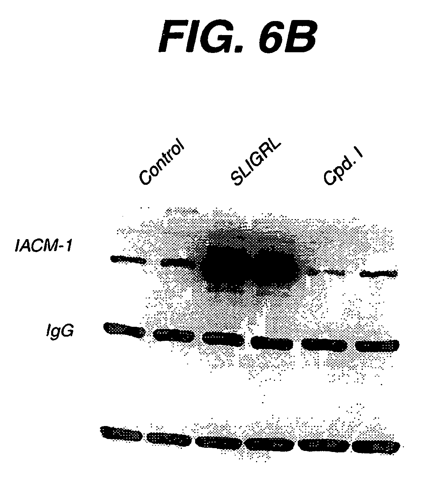

Immuno-precipitation and Western blotting experiments were performed, according to known methods, to assess the level of ICAM-1 protein in treated keratinocytes. As shown in Figure 6B, SLIGRL increased the level of ICAM-1 expression in these cells, whereas Compound I decreased the level of ICAM-1 expression.

### Example 10

### STI and Compound I Affect Chemotactic Cell Migration

Since ICAM-1 is involved in cell migration, cell migration towards a chemotactic peptide was studied in cells treated with Compound I and STI. Human PMN cells were placed in one side of a Boyden chamber, using standard techniques, and a chemotactic peptide (FMLP) was placed in the other chamber. Cells were allowed to migrate into the second chamber, and the number of migrating cells per field and distance of migration were calculated. The experiment was repeated with PMN cells pretreated with one of the following: 5 or 0.5 mg/ml STI, 1 or 0.1 µM Compound I, a buffer vehicle, and with and without chemotactic peptide. The number of cells per field that migrated the same distance as the untreated control was measured. These data are summarized in Table 5. Without the peptide and with no treatment, an average of 19 cells/field were migrating a distance of 80 microns. The addition of the chemotactic peptide resulted in 41 cells/field migrating 115 microns. Both compounds completely inhibited the migration of cells towards this peptide. In other words, no cells (or less than 5/field) were identified at 115 microns. This indicates that these inhibitors affect not only phagocytosis, but also cell migration. The ability to inhibit PMN migration is an important constituent of anti-inflammatory compounds.

**Table 5**

| Treatment | Cells/Field | Migration |
|---|---|---|
| Buffer | 19 | 80µ |
| FMLP | 41 | 115 |
| FMLP + Cpd I | <5 | 115 |
| FMLP+ STI | <5 | 115 |

### Example 11

### SLIGRL and STI Affect Human Skin Pigmentation

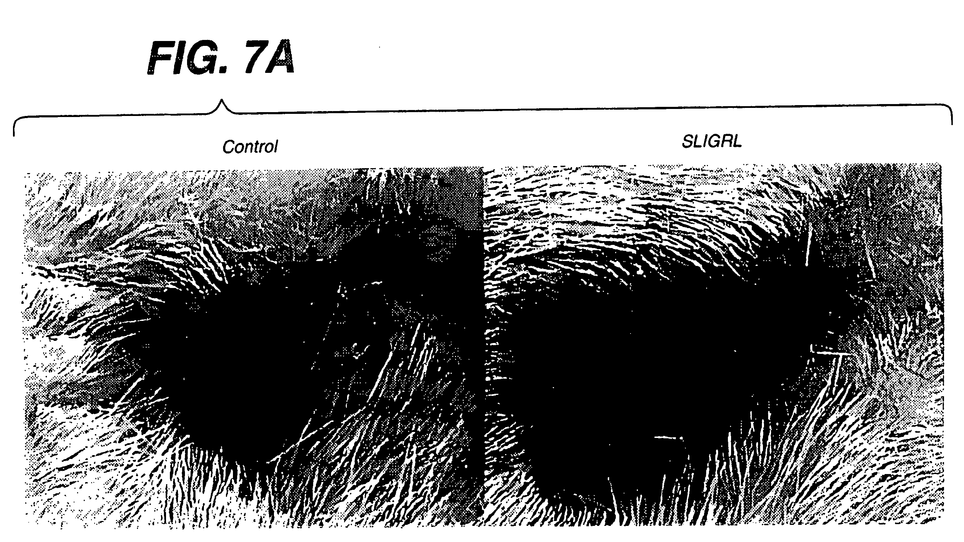

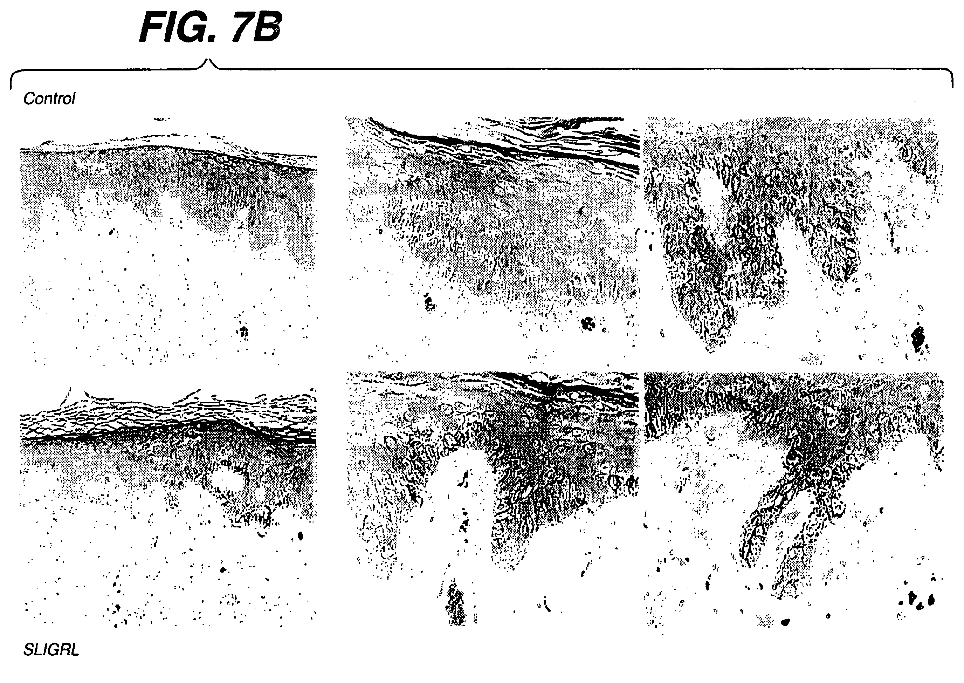

Human white facial skin samples were grafted on immuno-suppressed mice using standard techniques. About six weeks later grafts of the same individual, grafted on different mice, were treated with vehicle (ethanol: propylene glycol 70:30), or with 50 µM SLIGRL. Treatment was performed daily, 5 days/week. Darkening of the SLIGRL-treated grafts was visually observed during the last weeks of treatment (see Figure 7A). On day 66, the animals were sacrificed and their skins were analyzed histologically. F&M-stained sections revealed an increase in melanin in the SLIGRL-treated grafts, as shown in Figure 7B. This experiment demonstrates the ability to use SLIGRL on human skin to induce sunless tanning.

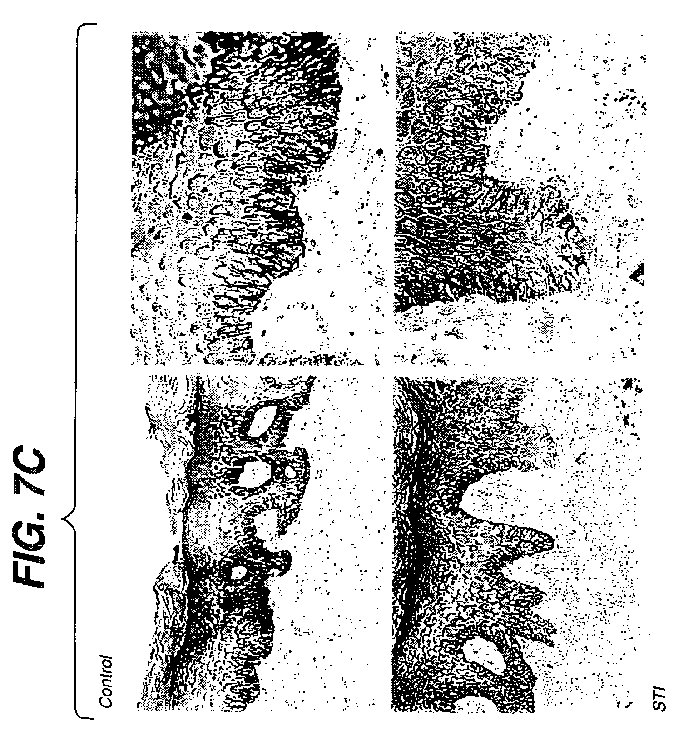

The same experiment was repeated with black human breast skin samples grafted on immuno-suppressed mice. Grafts of the same individual were treated with vehicle (GDL liposomes) or with 1% STI. GDL liposomes were prepared as set forth in Niemiec, et al., with the exception of the following changes: the non-ionic liposomal formulation contained glycerol dilaurate (Emulsynt GDL, ISP Van Dyk)/cholesterol (Croda)/polyoxyethylene-10-stearyl ether (Brij76, ICI)/polyoxyethylene-9-lauryl ether, at ratio of 37.5:12.5:33.3:16.7. Hepes buffer, 0.05M, pH 7.4 (Gibco-BRL of Gaithersburg, MD) was used as the aqueous phase in the preparation of the liposomes. Figure 7C shows F&M-stained skin sections from these grafts. This figure clearly demonstrates the ability of STI to reduce pigmentation in human skin.

### Example 12

### PAR-2 Effect on Phagocytosis and Migration Relates to Changes in Cell Shape

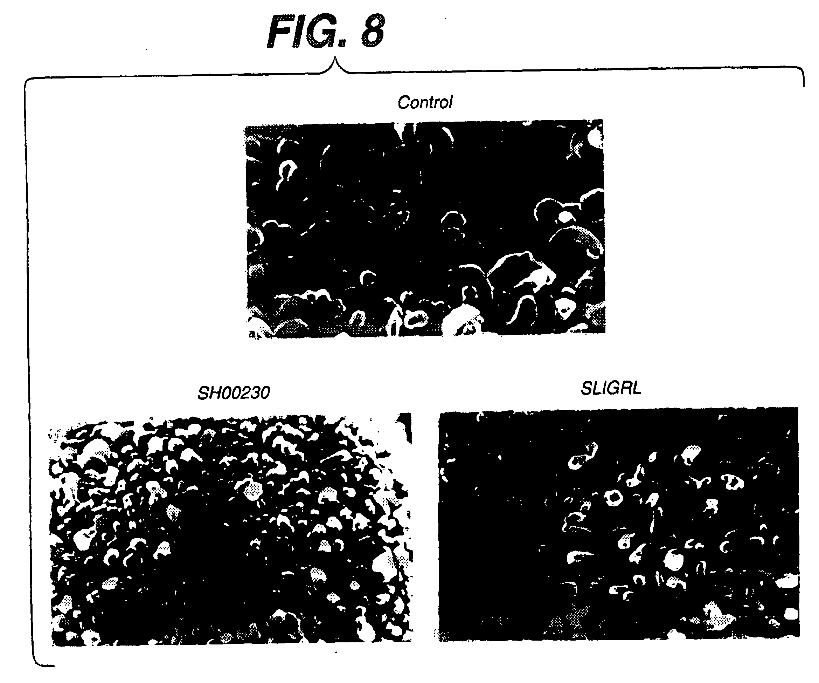

Both phagocytosis and cell migration involve changes in cell shape. Therefore, the effect of the PAR-2 pathway reagents on cell podia were studied using scanning electron microscopy. Keratinocytes were treated with Compound I (identified as "SH00230"), SLIGRL and a buffer vehicle for two days, and then processed for SEM using standard techniques. As shown in Figure 8, a dramatic change in the shape of cell podia is observed. Relative to the vehicle-treated controls, the Compound 1-treated cells have dramatically shorter podia. In other words, the cells' "fingers" are shorter and malformed, so they cannot grasp objects as well as can the control cells. The SLIGRL-treated samples demonstrated the opposite effect. Their podia were increased in number, were somewhat longer, and were much thinner. In other words, these cells have a greater probability for productive interaction with particles. Comparing these SEM pictures in Figure 8, it is clear that SLIGRL-treated cells have a greater ability to interact with particles, while Compound 1-treated cells are less suited for such a task.

### Example 13

### PAR-2 Affects Cytoskeletal Organization

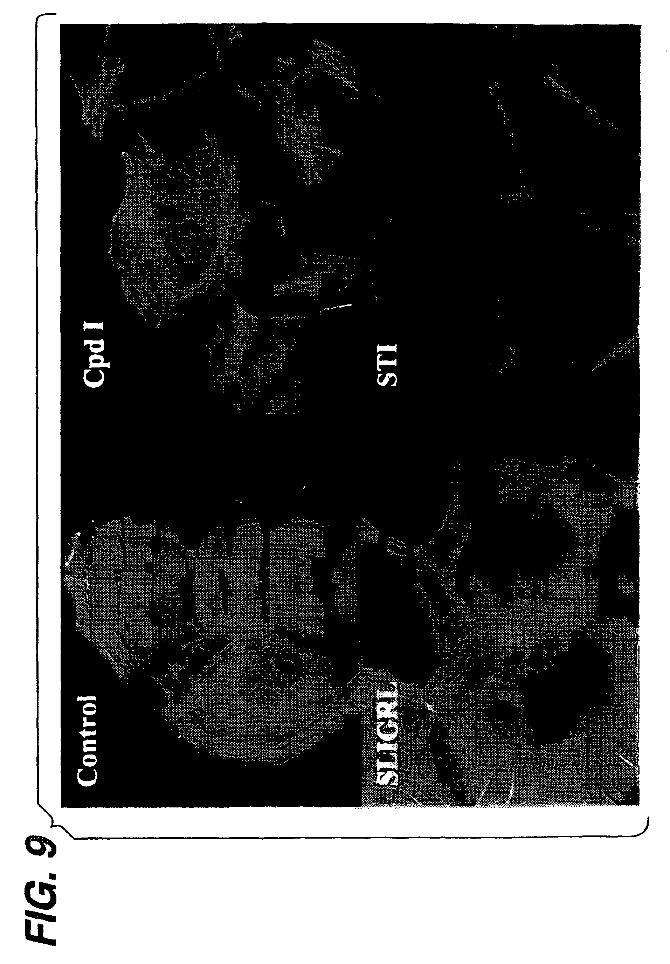

Changes in cell shape as demonstrated in Figure 8 require reorganization of cytoskeletal components. Therefore, the organization of F-actin filaments following PAR-2 modulation was tested. Keratinocytes were treated with Compound I (10 nM), STI (0.1 mg/ml), or with SLIGRL (5 µM) as described in Example 1, and stained for F-actin using standard techniques. Figure 9 shows dramatic changes in actin filaments organization following these treatments. SLIGRL treatment induced actin polymerization around the cell cortex, an area important in the control of cell movement and phagocytosis. STI and Compound I, on the contrary, reduced the ordered organization of the cell cortex, thereby reducing the cells' ability to regulate their movement and podia.

### Example 14

### ICAM-1 Modulation Affects Keratinocyte Phagocytosis

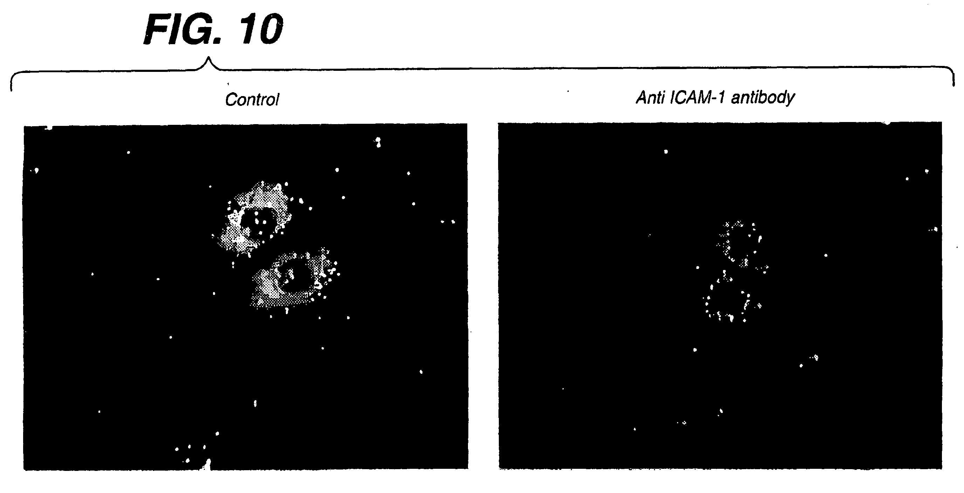

Keratinocytes were exposed to fluorescent microspheres (i.e. beads) as described in Example 1. These cells were pretreated with 50 pg/ml mouse antihuman ICAM-1 antibodies (from R&D Systems) for 16 hours, and then boosted again for four hours before incubating with the beads. As shown in Figure 10, blocking the surface ICAM-1 molecules on the keratinocytes results in reduced bead ingestion. This experiment therefore establishes a link between ICAM-1 and keratinocyte phagocytosis.

### Example 15

### STI/Liposome Formulation Can Lighten Human Age Spots

An individual with three age spots on the dorsum of her hand was treated for eight weeks, twice a day, as follows. The proximal age spot was treated with placebo, containing 20 mg/ml of liposomes. The median age spot was not treated. The distal age spot was treated with STI, 1%, in liposomes (20 mg/ml). GDL liposomes were prepared as set forth in Niemiec, et al., with the exception of the following changes. The non-ionic liposomal formulation contained glycerol dilaurate (Emulsynt GDL, ISP Van Dyk)/cholesterol (Croda)/polyoxyethylene-10-stearyl ether (Brij76, ICI)/polyoxyethylene-9-lauryl ether, as at ratio of 37.5:12.5:33.3:16.7. Hepes buffer, 0.05M, pH 7.4 (Gibco-BRL of Gaithersburg, MD) was used as the aqueous phase in the preparation of the liposomes. UV and visible light digital pictures were taken at times of 0, 4 and 8 weeks of treatment. L* (brightness) values were calculated from the images using Adobe Photoshop.

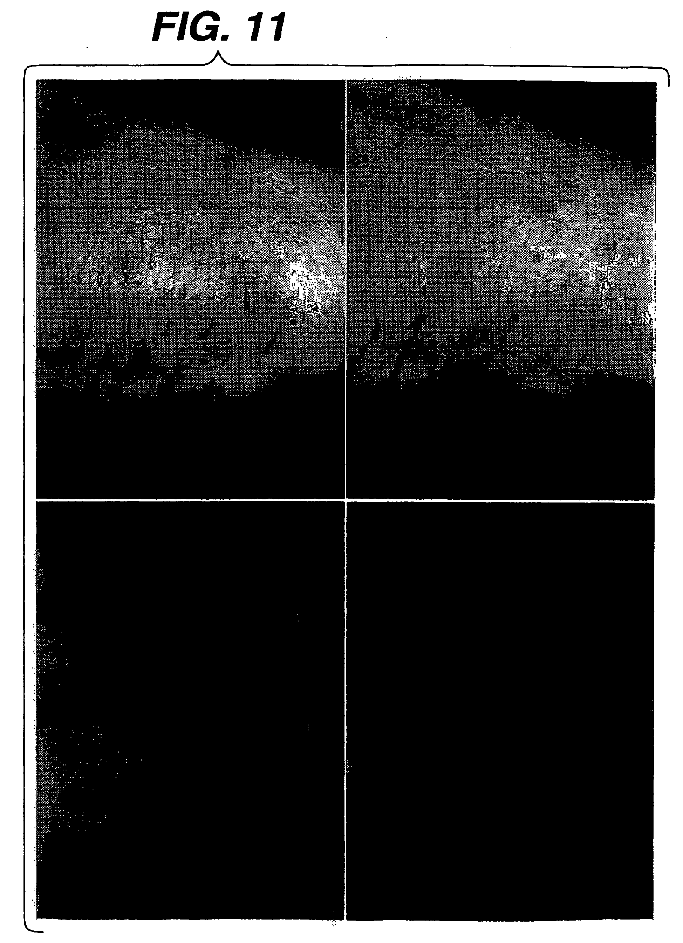

As shown in Figure 11, the age spot treated with STI became lighter following 8 weeks of treatment. Figure 11 is a composite of four pictures. The left panel is a visible light picture of the hand, before (upper) and after (lower) 8 weeks of treatment. At this orientation, the proximal age spot is placebo-treated, the median age spot is untreated, and the distal age spot is STI-treated. The right panel shows the same hand at the same time points, using UV-photography. UV light enables the visualization of pigment deeper in the skin, demonstrating that the STI whitening effect is not superficial. Figure 11 clearly demonstrates that the STI formulation lightened the distal age-spot. An increase of 15 L* units was calculated for this STI-treated spot, further demonstrating the ability of this treatment to lighten age spots.

### Example 16

### Phagocytosis-Reducing Compounds Analogous to Compound I

Certain compounds, and their pharmaceutically acceptable salts, such as those described in Costanzo, et al., "Potent Thrombin Inhibitors That Probe the S₁' Subsite: Tripeptide Transition State Analogues Based on a Heterocycle-Activated Carbonyl Group", J. Med. Chem., 1996, Vol. 39, pp. 3039-3043, behave as serine protease inhibitors (i.e. phagocytosis inhibitors), and have the following structural formula: 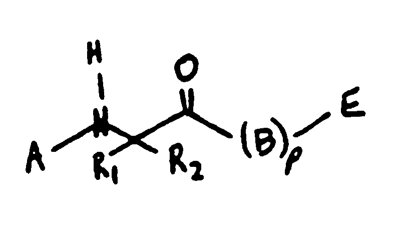 wherein:
A is selected from the group consisting of C₁₋₈alkyl, carboxyC₁₋₄alkyl, C₁₋₄alkoxycarbonylC₁₋₄alkyl, phenylC₁₋₄alkyl, substituted phenylC₁₋₄alkyl (where the phenyl substituents are independently selected from one or more of, C₁₋₄ alkyl, perfluoroC₁₋₄alkyl, C₁₋₄alkoxy, hydroxy, halo, amido, nitro, amino, C₁₋₄alkylamino, C₁₋₄dialkylamino, carboxy or C₁₋₄ alkoxycarbonyl), formyl, C₁₋₄alkoxycarbonyl, C₁₋₂alkylcarbonyl, phenylC₁₋₄alkoxycarbonyl, C3-7cycloalkylcarbonyl, phenylcarbonyl, substituted phenylcarbonyl (where the phenyl substituents are independently selected from one or more of C₁₋₄alkyl, perfluoroC₁₋₄alkyl, C₁₋₄alkoxy, hydroxy, halo, amido, nitro, amino, C₁₋₄alkylamino, C₁₋₄dialkylamino, carboxy or C₁₋₄ alkoxycarbonyl), C₁₋₄alkylsulfonyl, C₁₋₄alkoxysulfonyl, perfluoroC₁₋₄alkyl-sulfonyl, phenylsulfonyl, substituted phenylsulfonyl (where the phenyl substituents are independently selected from one or more of C₁₋₄alkyl, perfluoroC₁₋₄alkyl, C₁₋₄alkoxy, hydroxy, halo, amido, nitro, amino, C₁₋₄alkylamino, C₁₋₄dialkylamino, carboxy or C₁₋₄ alkoxycarbonyl), 10-camphorsulfonyl, phenylC₁₋₄alkysulfonyl, substituted phenylC₁₋₄alkylsulfonyl, C₁₋₄alkylsulfinyl, perfluroC_{1- 4}alkylsulfinyl, phenylsulfinyl, substituted phenylsulfinyl (where the phenyl substituents are independently selected from one or more of, C₁₋₄alkyl, perfluoroC₁₋₄alkyl, C₁₋₄alkoxy, hydroxy, halo, amido, nitro, amino, C₁₋₄alkylamino, C₁₋₄dialkylamino, carboxy or C₁₋₄alkoxycarbonyl), phenylC₁₋₄alkylsulfinyl, substituted phenylC₁₋₄alkylsulfinyl, 1-naphthylsulfonyl, 2-naphthylsulfonyl or substituted naphthylsulfonyl (where the naphthyl substituents are independently selected from one or more of, C₁₋₄alkyl, perfluoroC₁₋₄alkyl C₁₋₄alkoxy, hydroxy, halo, amido, nitro, amino, carboxy or C₁₋₄alkoxycarbonyl), 1-naphthylsulfinyl, 2-naphthylsulfinyl or substituted naphthylsulfinyl (where the naphthyl substituents are independently selected from one or more of, C₁₋₄alkyl, perfluoroC₁₋₄alkyl, C₁₋₄alkoxy, hydroxy, halo, amido, nitro, amino, C₁₋₄alkylamino, C₁₋₄dialkylamino, carboxy or C₁₋₄alkoxycarbonyl);
a D or L amino acid which is coupled as its carboxy terminus to the nitrogen depicted in the structure above and is selected from the group consisting of alanine, asparagine, 2-azetidinecarboxylic acid, glycine, N-C₁₋₈alkyglycine, proline, 1-amino-1-CycloC₃₋₈alkylcarboxylic acid, thiazolidine-4-carboxylic acid, 5,5-dimethylthiazolidine-4-carboxylic acid, oxazolidine-4-carboxylic acid, pipecolinic acid, valine, methionine, cysteine, serine, threonine, norleucine, leucine, tert-leucine, isoleucine, phenylalanine, 1-naphthalanine, 2-naphthalamine, 2-thienylalanine, 3-thienylalanine, [1,2,3,4]-tetrahydroisoquinoline- 1-carboxylic acid and [1,2,3,4,]-tetrahydroisoquinoline-2-caroboxylic acid
where the amino terminus of said amino acid is connected to a member selected form the group consisting of C₁₋₄alkyl, tetrazol-5yl-C₁₋₂alkyl, carboxyC₁₋₄alkyl, C₁₋₄alkoxycarbonylC₁₋₄alkyl, phenylC₁₋₄alkyl, substituted phenyl C₁₋₄ alkyl (where the phenyl substituents are independently selected from one or more of, C₁₋₄alkyl, perfluoroC₁₋₄alkyl, C₁₋₄alkoxy, hydroxy, halo, amido, nitro, amino, C₁₋₄alkylamino, C₁₋₄dialkylamino, carboxyl or C₁₋₄alkoxycarbonyl), 1,1-diphenylC₁₋₄alkyl, 3-phenyl-2-hydroxypropionyl, 2,2-diphenyl-1-hydroxyethylcarbonyl, [1,2,3,4]-tetrahydroisoquinoline-1-carbonyl, [1,2,3,4]-tetrahydroisoquinoline-3-carbonyl, 1-methylamino-1-cyclohexanecarbonyl, 1-hydroxy-1-cyclohexanecarbonyl, 1-hydroxy-1-phenylacetyl, 1-cyclohexyl-1-hydroxyacetyl, 3-phenyl-2-hydroxypropionyl, 3,3-diphenyl-2-hydroxypropionyl, 3-cyclohexyl-2-hydroxypropionyl, formyl, C₁₋₄alkoxycarbonyl, C₁₋₁₂alkylcarbonyl, perfluoroC₁₋₄alkyl, C₁₋₄alkylcarbonyl, phenylC₁₋₄alkylcarbonyl, substituted phenylC₁₋₄alkylcarbonyl (where the phenyl substituents are independently selected from one or more of, C₁₋₄alkyl, perfluoroC₁₋₄alkyl, C₁₋₄alkoxy, hydroxy, halo, amido, nitro, amino, C₁₋₄alkylamino, C₁₋₄dialkylamino, carboxy or C₁₋₄alkoxycarbonyl) 1, 1-diphenylC₁₋₄alkylcarbonyl, substituted 1,1-diphenylC₁₋₄alkylcarbonyl (where the phenyl substituents are independently selected from one or more of, C₁₋₄alkyl, perfluoro C₁₋₄alkyl, C₁₋₄alkoxy, hydroxy, halo, amido, nitro, amino, C₁₋₄alkylamino, C₁₋₄dialkylamino, carboxy or C₁₋₄ alkoxy-carbonyl), perfluoroC₁₋₄alkysulfonyl, C₁₋₄alkysulfonyl, C₁₋₄alkoxysulfonyl, phenysulfonyl, substituted phenylsulfonyl (where the phenyl substituents are independently selected from one or more of, C-1alkyl, perfluoroC₁₋₄alkylamino, C₁₋₄dialkylamino, carboxyl or C₁₋₄alkoxycarbonyl), 10-camphorsulfonyl, phenylC₁₋₄alkylsulfonyl, substituted phenylC₁₋₄alkylsufonyl, perfluroC₁₋₄alkysulfinyl, C-1-4alkysulfinyl, phenylsulfinyl, substituted phenysulfinyl (where the phenyl substituents are independently selected from one or more of, C₁₋₄alkyl, perfluoro C₁₋₄alkyl, C₁₋₄ alkoxy, hydroxy, halo, amido, nitro, amino, C₁₋₄ alkylamino, C₁₋₄ dialkylamino, carboxy or C₁₋₄ alkoxycarbonyl), 1-naphthysulfonyl, 1,2-naphthylsulfonyl, substituted naphthylsulfonyl (where the naphthyl substituents are independently selected from one or more of, C₁₋₄alkyl, perfluoroC₁₋₄alkyl, C₁₋₄alkoxy, hydroxy, halo, amido, nitro, amino, C₁₋₄alkylamino, C₁₋₄dialkylamino, carboxyl or C₁₋₄alkoxycarbonyl),1-naphthysulfinyl, 2-naphthysulfinyl, and substituted naphthylsulfinyl (where the naphthyl substituents are independently selected from one or more of, C₁₋₄alkyl, perfluoroC₁₋₄alkyl, C₁₋₄alkoxy, hydroxy, halo amido, nitro, amino, C₁₋₄alkylamino, C₁₋₄dialkylamino, carboxy or C₁₋₄alkoxycarbonyl);
or a polypeptide comprising two amino acids,
wherein the first amino acid is a D or L amino acid, bound via its carboxy terminus to the nitrogen depicted in Formula I and is selected from the group consisting of glycine, N-C₁₋₈alkylglycine, alanine, 2-azetidinecarboxylic acid, proline, thiazolidine-4-carboxylic acid, 5,5-dimethylthiazolidine-4-carboxylic acid, oxazolidine-4-carboxylic acid, 1-amino-1-cycloC₃₋₈ alkylcarboxylic acid, 3-hydroxyproline, 4-hydroxyproline, 3-(C₁₋₄alkoxy)proline, 4(C_{1- 4}alkoxy)proline, 3,4-dehydroproline, 2,2-dimethyl-4-thiazolidine carboxylic acid, 2,2-dimethyl-4-oxazolidine carboxylic acid, pipecolinic acid, valine, methionine, cysteine, asparagine, serine, threonine, leucine, tert-leucine, isoleucine, phenylalanine, 1-naphthalanine, 2-naphthalanine, 2-thienylalanine, 3-thienylalnine, [1,2,3,4]-tetrahydroisoquinoline-2-carboxylic acid, aspartic acid-4-C₁₋₄alkyl ester and glutamic acid 5-C₁₋₄alkyl ester and
wherein the second D or L amino acid, is bound to the amino terminus of said first amino acid, and is selected from the group consisting of phenylalanine, 4-benzoylphenylalanine, 4-carboxyphenylalanine, 4-(carboxyCl-2alkyl)phenylalanine, substituted phenylalanine (where the phenyl substituents are independently selected from one or more of C₁₋₄alkyl, perfluoroC₁₋₄alkyl, C₁₋₄alkoxy, hydroxy, halo, amido, nitro, amino, C₁₋₄alkylamino, C₁₋₄dialkylamino, carboxy or C₁₋₄alkoxycarbonyl), 3-benzothienylalanine, 4-biphenylalanine, homophenylalanine, octahydroindole-2-carboxylic acid, 2-pyridylalanine, 3-pyridylalanine, 4-thiazolylalanine, 2-thienylalanine, 3-(3-benzothienyl)alanine, 3-thienylalanine, tryptophan, tyrosine, asparagine, 3-tri-C₁₋₄alkylsilylalanine, cyclohexylglycine, diphenylglycine, phenylglycine, methionine sulfoxide, methionine sulfone, 2,2-dicyclohexylalanine, 2-(1-naphthylalanine), 2-(2-naphthylalanine), phenyl substituted phenylalanine (where the substituents are selected from C₁₋₄alkyl, perfluoroC₁₋₄alkyl, C₁₋₄ alkoxy, hydroxy, halo, amido, nitro, amino, C₁₋₄alkylamino, C₁₋₄dialkylamino, carboxy or C₁₋₄ alkoxycarbonyl), aspartic acid, aspartic acid-4-C₁₋₄alkyl ester, glutamic acid, glutamic acid-5-C_{1- 4}alkyl ester, cycloC₃₋₈alkylalanine, substituted cycloC₃₋₈alkylalanine (where the ring substituents are carboxy, C₁₋₄ alkyl ester, cycloC₃₋₈alkylalanine, substituted cycloC₃₋₈alkylalanine (where the ring substituents are carboxy, C₁₋₄alkylcarboxy, C₁₋₄alkoxycarbonyl or aminocarbonyl), 2,2-diphenylalanine and all alpha-C₁₋₅alkyl of all amino acid derivatives thereof, and
wherein the amino terminus of said second amino acid is unsubstituted or monosubstituted with a member of the group consisting of formyl, C₁₋₁₂ alkyl, tetrazol-5-yl C₁₋₂alkyl, carboxyC₁₋₈alkyl, carboalkoxyC₁₋₄alkyl, phenyl C₁₋₄alkyl, substituted phenylC₁₋₄alkyl (where the phenyl substituents or independently selected from one or more of, C₁₋₄alkyl, perfluoroC₁₋₄alkyl, C₁₋₄alkoxy, hydroxy, halo, amido, nitro, amino, C₁₋₄alkylamino, C₁₋₄dialkylamino, carboxy or C₁₋₄alkoxycarbonyl), 1,1-diphenylC₁₋₄alkyl, C₁₋₆alkoxycarbonyl, phenylC₁₋₆alkoxycarbonyl, C₁₋₂alkylcarbonyl, perfluoro C₁₋₄alkylcarbonyl, C₁₋₄alkylcarbonyl, phenyC₁₋₄alkylcarbonyl, substituted phenyC₁₋₄alkylcarbonyl(where the phenyl substituents are independently selected from one or more of C₁₋₄alkyl, perfluoro C₁₋₄alkyl, C₁₋₄ alkoxy, hydroxy, halo, amido, nitro, amino, C₁₋₄alkylamino, C₁₋₄dialkylamino, carboxy or C₁₋₄alkoxycarbonyl), 1,1-diphenylC₁₋₄alkyl, perfluoroC₁₋₄alkyl, C₁₋₄alkoxycarbonyl), 10-camphorsulfonyl, phenylC₁₋₄alkysulfonyl, substituted phenylC₁₋₄alkylsulfonyl, C₁₋₄alkysulfinyl, perfluoroC₁₋₄alkylsulfinyl, phenylsulfinyl, substituted phenylsulfinyl (where the phenyl substituents are independently selected from one or more of, C₁₋₄alkyl, perfluoroC₁₋₄alkyl, C₁₋₄alkoxy, hydroxy, halo, amido, nitro, amino, C₁₋₄alkylamino, C₁₋₄dialkylamino, carboxyl or C₁₋₄alkoxycarbonyl), phenylC₁₋₄alkylsulfinyl, substituted phenylC₁₋₄alkylsulfinyl, 1-naphthylsulfonyl, 2-naphthylsulfonyl, substituted naphthylsulfonyl (where the naphthyl substituent is selected from C₁₋₄alkyl, perfluoroC₁₋₄alkyl, C₁₋₄alkoxy, hydroxy, halo, amido, nitro, amino, C₁₋₄alkylamino, C₁₋₄dialkylamino, carboxy or C₁₋₄alkoxycarbonyl), 1-naphthyl-sulfinyl, 2-naphthylsulfinyl and substituted naphthylsulfinyl (where the naphthyl substituent is selected from C₁₋₄alkyl, perfluoroC₁₋₄alkyl, C₁₋₄alkoxy, hydroxy, halo, amido, nitro, amino, C₁₋₄alkylamino, C₁₋₄dialkylamino, carboxyl or C₁₋₄alkoxycarbonyl); R₁ is selected from the group consisting of hydrogen and alkyl; R₂ is selected from the group consisting of amino C₂₋₅alkyl, guanidinoC₂₋₅alkyl, C₁₋₄alkylguanidinoC₂₋₅alkyl, diC₁₋₄alkylguanidinoC₂₋₅alkyl, amidinoC₂₋₅alkyl, C₁₋₄alkylamidinoC₂₋₅alkyl, diC₁₋₄alkylamidinoC₂₋₅alkyl, C₁₋₃alkoxyC₂₋₅alkyl, phenyl, substituted phenyl (where the substituents are independently selected from one or more of amino, amidino, guanidino, C₁₋₄alkylamino, C₁₋₄dialkylamino, halogen, perfluoro C₁₋₄alkyl, C₁₋₄alkyl, C₁₋₃ alkoxy or nitro), benzyl, phenyl substituted benzyl (where the substituents are independently selected from one or more of, amino; amidino, guanidino, C₁₋₄alkylamino, C₁₋₄dialkylamino, halogen, perfluoroC₁₋₄alkyl, C1-4alkyl, C₁₋₃alkoxy or nitro), hydroxyC₂₋₅alkyl, C₁₋₅alkylaminoC₂₋₅alkyl, C₁₋₅dialkylaminoC₂₋₅alkyl, 4-aminocyclohexylC₀₋₂alkyl and C₁₋₅alkyl;
p is 0 or 1;
B is 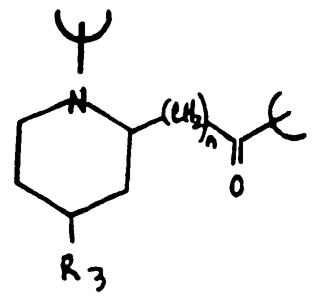
where n is 0-3, R₃ is H or C C₁₋₅alkyl and the carbonyl moiety of B is bound to E; E is a heterocycle selected from the group consisting of oxazolin-2-yl, oxazol-2-yl, thiazol-2-yl, thiazol-5-yl, thiazol-4-yl, thiazolin-2-yl, imidazol-2-yl, 4-oxo-2-quinoxalin-2yl, 2-pyridyl, 3-pyridyl, benzo[b]thiophen-2-yl, triazol-4-yl triazol-6-yl, pyrazol-2-yl, 4,5,6,7-tetrahydrobenzothiazol-2yl, naphtho[2,1-d]thiazol-2-yl, naphtho[1-2-d]thiazol-2-yl quinoxalin- 2-yl, isoquinolin-1-yl, isoquinolin-3-yl, benzo [b]furan-2-yl, pyrazin-2-yl, quinazolin-2-yl, isothiazol-5-yl, isothiazol-3-yl, purin-8-yl and a substituted heterocycle where the substituents are selected from C₁₋₄, perfluoro C₁₋₄alkyl,C₁₋₄alkoxy, hydroxy, halo, amido, nitro, amino, C₁₋₄alkylamino, C₁₋₄dialkylamino, carboxyl, C₁₋₄alkoxycarbonyl, hydroxy or phenylC₁₋₄ alkylaminocarbonyl, indol-2-yl, benzoxazol-2-yl, benzimidazol-2-yl and benzothiazol-2-yl.

## Claims

1. The use of a serine protease inhibitor for the manufacture of a medicament for treating a mammal afflicted with a disorder which is ameliorated by a decrease in phagocytosis or ICAM-1 expression in appropriate cells, said disorder being an immune system disorder, an inflammatory disorder, a disorder of the central nervous system, a skin disorder, a physical wound, a respiratory disorder, or restenosis.

2. The use of claim 1 wherein said disorder is an immune system disorder or an Inflammatory disorder.

3. The use of claim 1 or claim 2 wherein said serine protease inhibitor is a trypsin inhibitor.

4. The use of claim 3 wherein said trypsin inhibitor is soybean trypsin inhibitor.

5. Soybean trypsin inhibitor, for the treatment of a mammal afflicted with a disorder which is ameliorated by a decrease in phagocytosis or ICAM-1 expression in appropriate cells, said disorder being an immune system disorder, an inflammatory disorder, a disorder of the central nervous system, a skin disorder, a physical wound, a respiratory disorder, or restenosis.

## Patentansprüche

1. Verwendung eines Serinproteaseinhibitors zur Herstellung eines Medikaments zum Behandeln eines Säugetiers, das mit einer Störung geplagt ist, die durch eine Abnahme der Phagozytose oder der ICAM-1-Expression in passenden Zellen gelindert wird, wobei die Störung eine Störung des Immunsystems, eine entzündliche Störung, eine Störung des zentralen Nervensystems, eine Störung der Haut, eine physikalische Wunde, eine respiratorische Störung oder eine Restenose ist.

2. Verwendung nach Anspruch 1, wobei die Störung eine Störung des Immunsystems oder eine entzündliche Störung ist,

3. Verwendung nach Anspruch 1 oder 2, wobei der Serinproteaseinhibitor ein Trypsininhibitor ist.

4. Verwendung nach Anspruch 3, wobei der Trypsininhibitor ein Sojabohnentrypsininhibitor ist.

5. Sojabohnentrypsininhibitor zur Behandlung eines Säugetiers, das mit einer Störung geplagt ist, die durch eine Abnahme der Phagozytose oder der ICAM-1-Expression in passenden Zellen gelindert wird, wobei die Störung eine Störung des Immunsystems, eine entzündliche Störung, eine Störung des zentralen Nervensystems, eine Störung der Haut, eine physikalische Wunde, eine respiratorische Störung oder eine Restenose ist.

## Revendications

1. Utilisation d'un inhibiteur de sérine protéase pour la fabrication d'un médicament pour le traitement d'un mammifère affecté par un trouble qui est amélioré par une baisse de la phagocytose ou de l'expression de ICAM-1 dans des cellules appropriées, ledit trouble étant un trouble du système immunitaire, un trouble inflammatoire, un trouble du système nerveux central, un trouble cutanée, une lésion physique, un trouble respiratoire ou une resténose.

2. Utilisation selon la revendication 1, dans laquelle ledit trouble est un trouble du système immunitaire ou un trouble inflammatoire.

3. Utilisation selon la revendication 1 ou la revendication 2, dans laquelle ledit inhibiteur de sérine protéase est un inhibiteur de trypsine.

4. Utilisation selon la revendication 3, dans laquelle ledit inhibiteur de trypsine est un inhibiteur de trypsine du soja.

5. Inhibiteur de trypsine du soja pour le traitement d'un mammifère affecté par un trouble qui est amélioré par une baisse de la phagocytose ou de l'expression de ICAM-1 dans des cellules appropriées, ledit trouble étant un trouble du système immunitaire, un trouble inflammatoire, un trouble du système nerveux central, un trouble cutané, une lésion physique, un trouble respiratoire ou une resténose.
